# EUROPEAN PATENT SPECIFICATION

(11) **EP 1 379 689 B1**
(45) Date of publication and mention of the grant of the patent: **26.09.2012**
(21) Application number: 02713894.0
(22) Date of filing: 21.03.2002
(51) Int. Cl.: C12N 15/11, C07H 21/00, A61K 31/7088

(54) **OLIGONUCLEOTIDE CONJUGATES**
OLIGONUKLEOTIDKONJUGATE
CONJUGUES D'OLIGONUCLEOTIDE

(30) Priority: 23.03.2001 US 278322 P
(43) Date of publication of application: 14.01.2004
(73) Proprietor: GERON CORPORATION, Menlo Park, CA 94025 (US)
(72) Inventor: GRYAZNOV, Sergei, San Mateo, CA 94402 (US); PONGRACZ, Krisztina, Oakland, CA 94611 (US); TOLMAN, Richard, L., Los Altos, CA 94024 (US); MORIN, Gregg, B., Toronto, Ontario M9W7H4 (CA)
(74) Representative: Bassil, Nicholas Charles
(86) International application number: PCT/US2002/009138
(87) International publication number: WO 2002/077184

(56) References cited:
- WO-A-00/50279
- WO-A-01/18015
- US-A- 4 965 349
- US-A- 5 616 298
- US-A- 5 891 639
- MERGNY J-L ET AL: "TELOMERASE INHIBITORS BASED ON QUADRUPLEX LIGANDS SELECTED BY A FLUORESCENCE ASSAY" PROCEEDINGS OF THE NATIONAL ACADEMY OF SCIENCES OF USA, NATIONAL ACADEMY OF SCIENCE. WASHINGTON, US, vol. 98, no. 6, 13 March 2001 (2001-03-13), pages 3062-3067, XP001002239 ISSN: 0027-8424
- ASSELIN U ET AL: "NUCLEIC ACID-BINDING MOLECULES WITH HIGH AFFINITY AND BASE SEQUENCESPECIFICITY: INTERCALATING AGENTS COVALENTLY LINKED TO OLIGODEOXYNUCLEOTIDES", PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.81.11.3297, vol. 81, 1 June 1984 (1984-06-01), pages 3297-3301, XP002924014, ISSN: 0027-8424

## Description

### Field of the Invention

The present invention relates to oligonucleotides covalently attached to aromatic systems. More particularly, the present invention is directed to oligonucleotide conjugates that are designed to inhibit the activity of telomerase, an enzyme that is preferentially expressed in, and required for the growth of, cancer cells.

### Background of the Invention

### Oligonucleotide Chemistry

Nucleic acid polymer chemistry has played a crucial role in many developing technologies in the pharmaceutical, diagnostic, and analytical fields, and more particularly in the subfields of antisense and antigene therapeutics, combinatorial chemistry, branched DNA signal amplification, and array-based DNA diagnostics and analysis. Much of this chemistry has been directed to improving the binding strength, specificity, and nuclease resistance of natural nucleic acid polymers, such as DNA. Unfortunately, improvements in one property, such as nuclease resistance, often involve trade-offs against other properties, such as binding strength. Examples of such trade-offs abound: peptide nucleic acids (PNAs) display good nuclease resistance and binding strength, but have reduced cellular uptake in test cultures (e.g. Hanvey et al., Science, 258:1481-1485, 1992); phosphorothioates display good nuclease resistance and solubility, but are typically synthesized as P-chiral mixtures and display several sequence-non-specific biological effects (e.g. Stein et al., Science, 261:1004-1012, 1993); methylphosphonates display good nuclease resistance and cellular uptake, but are also typically synthesized as P-chiral mixtures and have reduced duplex stability, and so on.

Recently, a new class of oligonucleotide analog has been developed having so-called N3'→P5' phosphoramidate internucleoside linkages, which display favorable nucleic acid binding properties, nuclease resistance, and water solubility (Gryaznov and Letsinger, Nucleic Acids Research, 20:3403-3409, 1992; Chen et al., Nucleic Acids Research, 23:2661-2668, 1995; Gryaznov et al., Proc. Natl. Acad. Sci., 92:5798-5802, 1995; and Gryaznov et al., J. Am. Chem. Soc., 116:3143-3144, 1994). Uniformly modified phosphoramidate compounds contain a 3'-amino group at each of the 2'-deoxyfuranose nucleoside residues replacing a 3'-oxygen atom. The synthesis and properties of oligonucleotide N3'→P5' phosphoramidates are also described in U.S. Patent Nos. 5,591,607; 5,599,922; 5,726,297; and 5,824,793.

Oligonucleotides conjugated to a signal generating system have been used as tools in diagnostic applications, such as fluorescent in situ hybridization (FISH). For example, specific microorganism (U.S. Patent No. 5,776,694 or telomerase-expressing cells (U.S. Patent No. 5,891,639 were identified by labeling nucleic acids that were complementary to sequences unique to the target organism or the telomerase enzyme, and then contacting the probe with the target. Once the probe had formed a hybrid with the target, the hybrid was detected by activating the signal generating system that was bound to the probe. In another use, labeled probes were used in DNA microarray experiments (See U.S. Patent No. 6,040,138 to). Typically, probes from a biological sample were amplified in the presence of nucleotides that had been coupled to a reporter group, such as a fluorescent label, thereby creating labeled probes. The labeled probes were then incubated with the microarrays so that the probe sequences hybridized to the complementary sequences immobilized on the microarray. A scanner was then used to determine the levels and patterns of fluorescence. The present invention relates to a new class of oligonucleotide conjugates having therapeutic uses.

### Telomerase

Telomerase is a ribonucleoprotein that catalyzes the addition of telomeric repeat sequences to chromosome ends. See Blackburn, 1992, Annu. Rev. Biochem., 61:113-129. There is an extensive body of literature describing the connection between telomeres, telomerase, cellular senescence and cancer (for a general review, see Oncogene, volume 21, January 2002, which is an issue focused on telomerase). Telomerase has therefore been identified as an excellent target for cancer therapeutic agents (see Lichsteiner et al., Annals New York Acad. Sci. 886:1-11, 1999)

Genes encoding both the protein and RNA components of human telomerase have been cloned and sequenced (See U. S. Patent Nos. 6,261,836 and 5,583,016, respectively) and much effort has been spent in the search telomerase inhibitors. Telomerase inhibitors identified to date include small molecule compounds and oligonucleotides. By way of example, WO01/18015 describes the use of oligonucleotides that comprise N3'→P5' thio-phosphoramidate internucleoside linkages and which are complementary to the sequence of the human telomerase RNA component to inhibit telomerase activity.

### Summary of the Invention

The compositions and methods of the present invention relate to compounds comprising an oligonucleotide and at least one covalently linked aromatic system as defined in the claims. The sequence of the oligonucleotide as defined in the claims is selected to be complementary to the RNA component of telomerase, and the covalently linked aromatic system is a polyaromatic hydrocarbon, and preferably a fluorophore as defined in the claims. The aromatic group is typically, although not necessarily, covalently attached to either the 3'- position or the 5'-position of the oligonucleotide, and in particular embodiments aromatic groups may be attached to each of the 3'- and 5'- positions. In their simplest form, the compounds of the invention as defined in the claims be represented by the formula:

(A-L-)ₙ-O

wherein A comprises a heteroaromatic group, L is a linker group, O is an oligonucleotide and n is an integer between 1 and 1+m where m is the total number of nucleosides comprising the oligonucleotide wherein the compound inhibits telomerase activity. In preferred embodiments, n=1 or 2.

The oligonucleotide components of the compounds comprise nucleosides as defined in the claims and the intersubunit linkages between the nucleosides can be formed using N3'→P5' phosphoramidate or N3'→P5' thiophosphoramidate linkages. The sequence of the oligonucleotide O is selected such that it is complementary to the template region of the RNA component of telomerase.

The moiety A as defined in the claims comprises a polyaromatic substituted or unsubstituted hydrocarbon, with fluorophores being particularly preferred. Examples of suitable aromatic systems include chromophores and fluorophores selected from fluoresceins, rhodamines, and acridines as defined in the claims. The aromatic systems may be attached to the oligonucleotide at the 3'-position, at the 5'-position, on the intersubunit linkage, on the base, or combinations thereof. In compounds having more than one aromatic system attached to the oligonucleotide, each conjugate and each linker may be independently chosen.

The A group is covalently attached to the oligonucleotide directly through a linker, L as defined in the claims.

The oligonucleotide conjugate compounds of the present invention may be used in methods to inhibit telomerase enzymatic activity. Such methods comprise contacting the telomerase enzyme with a compound of the invention. Also provided herein are pharmaceutical compositions comprising an oligonucleotide conjugate according to the invention formulated in a pharmaceutically acceptable excipient.

An oligonucleotide conjugate that is illustrative of the compounds of the present invention is shown schematically below, with the three components A, L and O shown as bracketed. In this instance, the two 3'-nucleosides of the oligonucleotide (O) are shown, and the aromatic group (A) fluorescein is linked to the 3'-end of the oligonucleotide through a linker (L) that is a thiourea group. B represents the nucleoside bases, and the oligonucleotide linkages shown are N3'→P5' thio-phosphoramidate linkages.

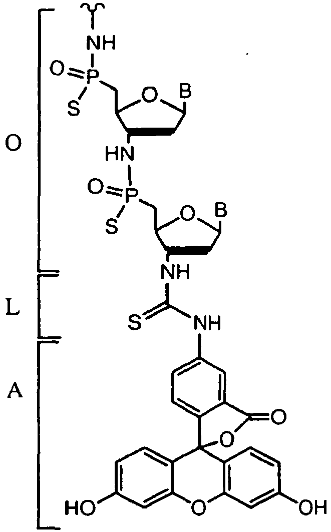

### Brief Description of the Drawings

FIGURE 1 shows the structure of an exemplary oligonucleotide conjugate of the invention wherein the polynucleotide AGG (SEQ ID NO.: 1) having phosphoramidate internucleoside linkages is attached to fluorescein via a thiourea group.
FIGURE 2 shows the structures of exemplary aromatic systems having linker groups attached to them.

### Detailed Description

### A. Definitions

An "alkyl group" refers to an alkyl or substituted alkyl group having 1 to 20 carbon atoms, such as methyl, ethyl, propyl, and the like. Lower alkyl typically refers to C₁ to C₅. Intermediate alkyl typically refers to C₆ to C₁₀.

An "aryl group" refers to an aromatic ring group having 5-20 carbon atoms, such as phenyl, naphthyl, anthryl, or substituted aryl groups, such as, alkyl- or aryl-substitutions like tolyl, ethylphenyl, biphenylyl, etc. Also included are heterocyclic aromatic ring groups having one or more nitrogen, oxygen, or sulfur atoms in the ring. "Oligonucleotide" refers to nucleoside subunit polymers having between about 2 and about 50 contiguous subunits. The nucleoside subunits can be joined by a variety of intersubunit linkages, including, but not limited to, phosphodiester, phosphotriester, methylphosphonate, P3'→N5' phosphoramidate, N3'→P5' phosphoramidate, N3'→P5' thio-phosphoramidate, and phosphorothioate linkages.

Further, "oligonucleotides" includes modifications, known to one skilled in the art, to the sugar backbone (e.g., ribose or deoxyribose subunits), the sugar (e.g., 2' substitutions), the base, and the 3' and 5' termini. The term "polynucleotide", as used herein, has the same meaning as "oligonucleotide" and is used interchangeably with "polynucleotide". In embodiments where the oligonucleotide moiety includes a plurality of intersubunit linkages, each linkage may be formed using the same chemistry or a mixture of linkage chemistries may be used.

Whenever an oligonucleotide is represented by a sequence of letters, such as "ATGUCCTG," it will be understood that the nucleotides are in 5' → 3' order from left to right. Representation of the base sequence of the oligonucleotide in this manner does not imply the use of any particular type of internucleoside subunit in the oligonucleotide.

As used herein, "nucleoside" includes the natural nucleosides, including 2'-deoxy and 2'-hydroxyl forms, e.g. as described in Komberg and Baker, DNA Replication, 2nd Ed. (Freeman, San Francisco, 1992), and analogs. "Analogs" in reference to nucleosides includes synthetic nucleosides having modified base moieties and/or modified sugar moieties, e.g. described generally by Scheit, Nucleotide Analogs (John Wiley, New York, 1980). Such analogs include synthetic nucleosides designed to enhance binding properties, e.g. stability, specificity, or the like, such as disclosed by Uhlmann and Peyman (Chemical Reviews, 90:543-584, 1990).

A "base" is defined herein to include (i) typical DNA and RNA bases (uracil, thymine, adenine, guanine, and cytosine), and (ii) modified bases or base analogs (e.g., 5-methylcytosine, 5-bromouracil, or inosine). A base analog is a chemical whose molecular structure mimics that of a typical DNA or RNA base.

As used herein, "pyrimidine" means the pyrimidines occurring in natural nucleosides, including cytosine, thymine, and uracil, and common analogs thereof, such as those containing oxy, methyl, propynyl, methoxy, hydroxyl, amino, thio, halo, and like, substituents. The term as used herein further includes pyrimidines with common protection groups attached, such as N₄-benzoylcytosine. Further common pyrimidine protection groups are disclosed by Beaucage and Iyer (Tetrahedron 48:223-2311, 1992).

As used herein, "purine" means the purines occurring in natural nucleosides, including adenine, guanine, and hypoxanthine, and common analogs thereof, such as those containing oxy, methyl, propynyl, methoxy, hydroxyl, amino, thio, halo, and like, substituents. The term as used herein further includes purines with common protection groups attached, such as N₂-benzoylguanine, N₂-isobutyrylguanine, N₆-benzoyladenine, and the like. Further common purine protection groups are disclosed by Beaucage and Iyer (cited above).

As used herein, the term "protected" as a component of a chemical name refers to art-recognized protection groups for a particular moiety of a compound, e.g. "5'-protected-hydroxyl" in reference to a nucleoside includes triphenylmethyl (i.e., trityl), p-anisyldiphenylmethyl (i.e., monomethoxytrityl or MMT), di-p-anisylphenylmethyl (i.e., dimethoxytrityl or DMT), and the like. Art-recognized protection groups include those described in the following references: Gait, editor, Oligonucleotide Synthesis: A Practical Approach (IRL Press, Oxford, 1984); Amarnath and Broom, Chemical Reviews, 77:183-217, 1977; Pon et al., Biotechniques, 6:768-775, 1988; Ohtsuka et al., Nucleic Acids Research, 10:6553-6570, 1982; Eckstein, editor, Oligonucleotides and Analogues: A Practical Approach (IRL Press, Oxford, 1991), Greene and Wuts, Protective Groups in Organic Synthesis, Second Edition, (John Wiley & Sons, New York, 1991), Narang, editor, Synthesis and Applications of DNA and RNA (Academic Press, New York, 1987), Beaucage and Iyer (cited above), and like references.

The term "halogen" or "halo" is used in its conventional sense to refer to a chloro, bromo, fluoro or iodo substituent. In the compounds described and claimed herein, halogen substituents are generally fluoro, bromo, or chloro, preferably fluoro or chloro.

### B. Design and Synthesis of the Oligonucleotide Conjugates

The present invention is directed to compounds as defined in the claims having the formula:

(A-L-)ₙ-O

wherein A is an aromatic group, L is a linker, O is an oligonucleotide, n is an integer between 1 and 1+m where m is the total number of nucleosides comprising the oligonucleotide. Design of the compounds therefore requires the selection of three entities, A, L and O and the determination of the structural linkages between these entities.

### Selection of O

The oligonucleotide sequence of O as defined in the claims is selected such that it is complementary to the RNA sequence of the target telomerase, e. g., human telomerase. The RNA component of human telomerase has been sequenced (see Feng et al., Science 269 (5228), 1236-1241, 1995, sequence data also available on GenBank, Accession No. U86046). Within the human telomerase RNA ("hTR") sequence is a region identified as the "template region" that functions to specify the sequence of the telomeric repeats that telomerase adds to the chromosome ends and is this essential to the activity of the enzyme (see Chen et al., Cell 100:503-514, 2000; Kim et al, Proc. Nat'l. Acad. Sci, USA 98(14): 7982-7987, 2001). The template region is an 11 base region of sequence 5'-CUAACCCUAAC-3' (SEQ ID: 2) that has therefore been identified as a particularly suitable target for inhibitory oligonucleotides. Accordingly, the oligonucleotide sequence of the O component of the oligonucleotide conjugates is a sequence from this template. The sequence selected is preferably exactly complementary to the corresponding hTR sequence. While mismatches may be tolerated in certain instances, they are expected to decrease the specificity and activity of the resultant oligonucleotide conjugate. Preferably the full length of the oligonucleotide O is selected to be complementary to the corresponding hTR sequence.

Oligonucleotides of shorter lengths are preferred since shorter molecules are.(a) more easily synthesized and may be produced at a lower cost and (b) likely to be more bio-available. Thus, in preferred embodiments the length of the oligonucleotide O components is from 4-8 nucleosides. The examples of oligonucleotide conjugates presented below show that lengths of O of 4-8 nucleosides have potent telomerase inhibition activity. One of skill in the art will appreciate that the selection of the sequence and length of the O component may also be affected by the selection of the L and A components of the conjugate. Simple experimentation using the standard telomerase activity assays described herein will facilitate selection of the optimal combinations. The inventive telomerase inhibitor modified oligonucleotides have a consecutive base sequence that includes a region that is complementary to all or part of the template region of telomerase RNA selected from the group consisting of:
TAGGGTTAGACAA (SEQ ID: 3); GTTAGGGTTAG (SEQ ID: 4); AGGGTTAG (SEQ ID: 5); GGGTTAG (SEQ ID: 6); GTTAGG (SEQ ID_{:} 7); TTAGGG (SEQ ID_{:} 8); AGGG (SEQ ID_{:} 10); GGGTTA (SEQ ID: 11); TGAGTG (SEQ ID: 12); and GTAGGT (SEQ ID: 13).
The inter-nucleoside linkages used in the synthesis of the O component may be N3'→P5' phosphoramidate or N3'→N5' thio-phosphoramidate linkages.

Oligonucleotides having at least one N3'→P5' phosphoramidate or N3'→P5' thio-phosphoramidate linkage may have characteristics that provide advantages in the therapeutic context. Exemplary oligonucleotide linkages may be represented by the formula:

3'-[-Z₆-P(=O)(-Z₇R)-O-]-5'

wherein Z₆ is O or NH; Z₇ is O or S; and R is selected from the group consisting of hydrogen, alkyl, aryl and salts thereof.

Methods for synthesizing oligonucleotides suitable for incorporation into the conjugates described herein are well known in the art. By way of example, oligonucleotide N3'→P5' thio-phosphoramidates for use in the invention may be synthesized using the phosphoramidite transfer methodology of Pongracz et al., Tet. Let. 40: 7661-7664, 1999).. This synthetic strategy employs 3'-NH-trityl-protected 3'-aminonucleoside 5'-O-cyanoethyl-N,N-diisopropylaminophosphor-amidites which are processed through the steps of 1) detritylation, 2) coupling; 3) capping; 4) sulfurization. To achieve a step-wise sulfurization of the internucleaside phosphoramidite group formed after the coupling step, sulfurizing agents such as elemental sulfur S₈ or PADS (diphenylacetyldisulfide) or the commonly used Beaucage reagent -3H-1,2-benzodithiol-3-one 1,1 dioxide (Iyer et al., J. Organic chemistry 55:4693-4699, 1990) can be used. The oligonucleotide syntheses may be performed (1 µmole synthesis scale) with a 1% solution of Beaucage reagent in anhydrous acetonitrile or 15% S₈ in CS₂/Et₃N, 99/1 (vol/vol) as the sulfurizing agent.

Chimeric N3'→P5' phosphoramidate-phosphorthioamidate oligonucleotides can be made by using an oxidation step(s) after the coupling step, which results in formation of a phosphoramidate internucleoside group. Similarly, phosphodiester-phosphorthioamidates can be made by using 5'-phosphoramidite-3'-O-DMTr-protected nucleotides as monomeric building blocks. These synthetic approaches are known in the art.

### Selection of A

It is believed that the conjugates of the present invention bind to telomerase in two aspects, thereby inhibiting function of the enzyme. The first aspect of the binding is the base-pair complementarity between the nucleosides of hTR and the nucleosides of the oligonucleotide (O) component of the conjugate. The second aspect of the binding is the interaction between the telomerase protein component and the aromatic-containing (A) component of the conjugate.
Substituents comprising polyaromatic hydrocarbons are used according to the claims and, in particular embodiments, fluorophores are shown to be particularly effective. Classes of suitable aromatic systems include chromophores and fluorophores selected from fluoresceins, rhodamines and acridines as defined in the claims.

As with the selection of the oligonucleotide (O) component, the activity of a selected aromatic (A) component may readily be determined using standard telomerase activity assays.
Fluorescein, having the formula: 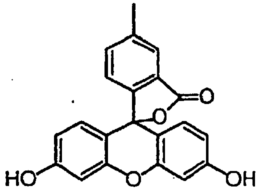 N,N'-tetramethylrhodamine, having the formula:

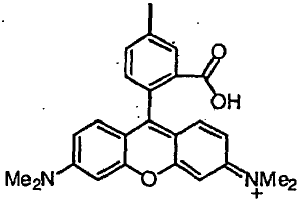

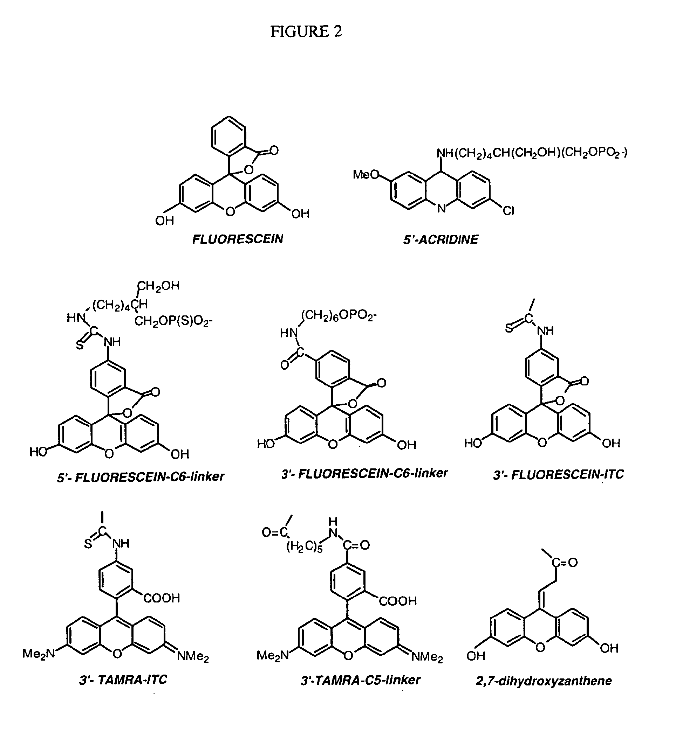

Other aromatic substituents (shown conjugated with linker (L) groups) are depicted in Fig. 2. Aromatic substituents that may be employed in the oligonucleotide conjugates of the present invention may be purchased from chemical reagent suppliers or synthesized using standard chemical synthesis procedures.

### Selection of L

The linkage between the O and A components incorporates a linker sequence. Such a sequence may serve not only to facilitate the chemical conjugation of the two moieties, but may also serve to increase the activity of the conjugate by conferring flexibility and thereby enhancing the available to each moiety to bind to its target on the telomerase enzyme. A variety of linker groups may be utilized, depending on the selection of the A component as defined in the claims. For the purposes of reference linkers are the structures: 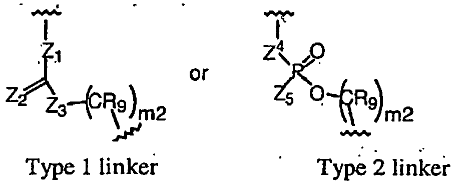 Wherein:
Z₁, Z₂, and Z₃ are independently selected from the group consisting of O, S, and NR₄, wherein R₄ is H or lower alkyl and Z₁ and Z₃ can be direct bonds;
Z₄ is O or NH;
Z₅ is OR', SR', or methyl wherein R' is selected from the group consisting of hydrogen, alkyl, aryl and salts thereof;
R₉ is H, halogen or lower alkyl, and m2 is an integer from 0 to 10.

One example of a type 1 linker is the thiourea linker in which: Z₁ = NH; Z₂ = S; Z₃ = NH; and m2 = 0 (i.e., there is no carbon between the Z₃ = N substituent and the A moiety. A type 1 thiourea linker is shown in the structure depicted in the Summary section above. Another way of representing the structure of oligonucleotide conjugates having a type 2 linker is as follows: 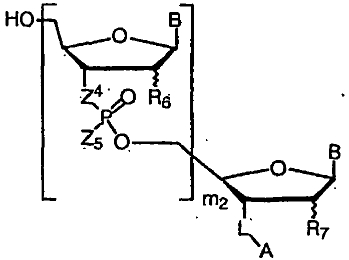 wherein each B is a base independently selected to be a purine or pyrimidine or an analog thereof such as uracil, thymine, adenine, guanine, cytosine, 5-methylcytosine, 5-bromouracil and inosine, A is the aromatic moiety and L is a type 2 linker. In this arrangement, it is apparent that the definition of the linker structure (type 2 linker above) incorporates components of the internucleoside linkage. Reference to a "C5" or "C6" linker refers to a type 2 linker in which m2 = 5 or 6.

### Arrangement of Components

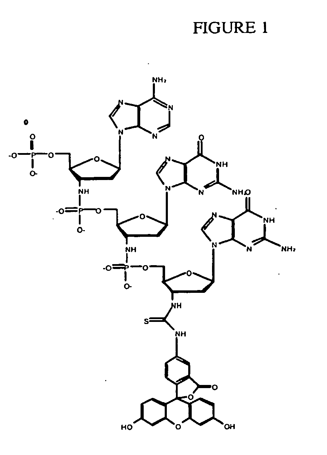

As implied by the structure (A-L-)ₙ-O, wherein A is an aromatic group, L is a linker, O is an oligonucleotide, n is an integer between 1 and 1+m where m is the total number of nucleosides comprising the oligonucleotide, multiple A substituents may be conjugated to the oligonucleotide. Moreover, the A substituents may be attached to the oligonucleotide at the 3'-position, at the 5'-position, on the intersubunit linkage, on the base, or combinations thereof. However, in typical embodiments, one or two A substituents are used (i.e., n=1 or 2), and these are attached thorough L to the 3'-position or the 5'-position of the oligonucleotide O. Figure 1 illustrates an example in which the A substituent is fluorescein attached to the 3'-position of the oligonucleotide. Where two or more A substituents are utilized, the A substituents are independently selected. As with the selection of all of the components, the design of the conjugate can readily be tested by performing standard telomerase activity assays as described below.

### Synthesis of the Oligonucleotide Conjugates

The aromatic substituent can be covalently linked to the oligonucleotide using standard chemical syntheses. Typically, the nucleophilic sites on the oligonucleotide are first protected and the desired site of attachment is selectively deprotected. The oligonucleotide is then reacted with, for example in the case of the aromatic substituent being fluorescein, fluorescein containing a reactive group such as isothiocyanate, isocyanate, monochlorotriazine, dichlorotriazine, mono- or di-halogen substituted pyridine, mono- or di-halogen substituted diazine, maleimide, aziridine, sulfonyl halide, acid halide, hydroxysuccinimide ester, hydroxysulfosuccinimide ester, imido ester, hydrazine, azidonitrophenyl, azide, 3-(2-pyridyl dithio)-proprionamide, glyoxal or aldehyde, to yield a modified oligonucleotide in which fluorescein is bound to the oligonucleotide, preferably through a thiourea or an amide group, as shown in the scheme below:

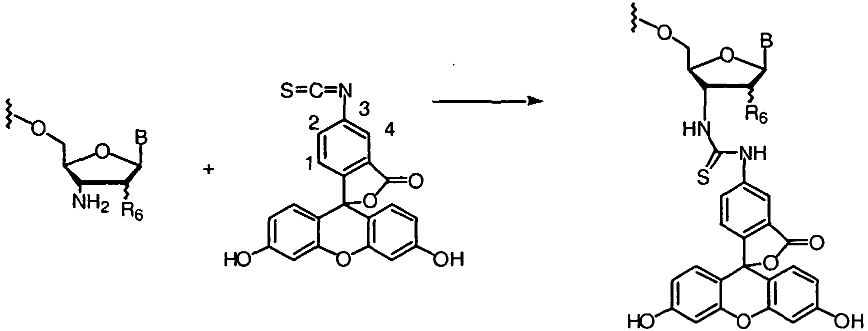

The linker L may be joined to the aromatic system A at position 1, 2, 3, or 4. Preferably, L is joined to A at the 3 position.

Alternatively, the linker can first be converted into a phosphoramidite followed by coupling to the oligonucleotide, as described in U.S. Patent No. 5,583,236 to Brush. Typically, when the aromatic system is fluorescein, commercially available fluorescein 3-isothiocyanate diacetate is reacted sequentially with aminopenta-6-ol and then a phosphitylating reagent to yield a phosphoramidite linked to fluorescein via a five carbon chain and a thiourea group, as shown below: 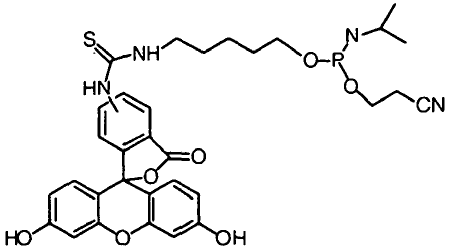 Some examples of a linker L joined to a suitable aromatic system A are illustrated in Figure 2.

### Telomerase Inhibition Assays

The conjugates of the present invention may be used to inhibit or reduce telomerase enzyme activity and/or proliferation of cells having telomerase activity. In these contexts, inhibition or reduction of the enzyme activity or cell proliferation refer to a lower level of the measured activity relative to a control experiment in which the enzyme or cells are not treated with the conjugate. In particular embodiments, the inhibition or reduction in the measured activity is at least a 10% reduction or inhibition. One of skill in the art will appreciate that reduction or inhibition of the measured activity of at least 20%, 50%, 75%, 90% or 100% may be preferred for particular applications.

Methods for measuring telomerase activity, and the use of such methods to determine the telomerase inhibitory activity of compounds are well known. For example, the TRAP assay is a standard assay method for measuring telomerase activity in a cell extract system and has been widely used in the search for telomerase inhibiting compounds (Kim et al., Science 266:2011, 1997; Weinrich et al., Nature Genetics 17:498, 1997). The TRAP assay measures the amount of radioactive nucleotides incorporated into elongation products (polynucleotides) formed by nucleotide addition to a telomerase substrate or primer. The radioactivity incorporated can be measured as the intensity of a band on a detection screen (e.g. a Phosphorimager screen) exposed to a gel on which the radioactive products are separated. The TRAP assay is also described in detail in U.S. Patent Nos. 5,629,154, 5,837,453 and 5,863,726, and its use in testing the activity of telomerase inhibitory compounds is described in various publications including WO 01/18015. In addition, the following kits are available commercially for research purposes for measuring telomerase activity: TRAPeze® XK Telomerase Detection Kit (Cat. s7707; Intergen Co., Purchase NY); and TeloTAGGG Telomerase PCR ELISA plus (Cat. 2,013,89; Roche Diagnostics, Indianapolis IN).

Typically, the ability of the conjugates of the invention to inhibit telomerase activity will first be confirmed by performing a biochemical telomerase activity assay, such as the TRAP assay, and comparing the results obtained in the presence of the conjugate with results obtained from a control experiment performed without the conjugate. Such data permit the calculation of an IC₅₀ value for the conjugate (the concentration of the test compound at which the observed activity for a sample preparation is observed to fall one-half of its original or a control value). Using such methods, IC₅₀ values for several of the oligonucleotide conjugates of the present invention were determined, and found to be below 10 µM.

In addition, the specificity of the oligonucleotide conjugates for telomerase RNA can be determined by performing hybridization tests with and comparing their activity (IC₅₀) with respect to telomerase and to other enzymes known to have essential RNA components, such as ribonuclease P. Compounds having lower IC₅₀ values for telomerase as compared to the IC₅₀ values toward the other enzymes being screened are said to possess specificity for telomerase. Having confirmed that a particular conjugate is an effective telomerase inhibitor using a biochemical assay, it may then be desirable to ascertain the ability of the conjugate to inhibit telomerase activity in cells. Oligonucleotide conjugates that effectively inhibit telomerase activity in cells will, like other known telomerase-inhibiting compounds, induce crisis in telomerase-positive cell lines. Cell lines that are suitable for such assays include HME50-5E human breast epithelial cells, and the ovarian tumor cell lines OVCAR-5 and SK-OV-3. Importantly however, in normal human cells used as a control, such as BJ cells of fibroblast origin, the observed reduction in telomere length is expected to be no different from untreated cells. In selecting an oligonucleotide conjugate of the invention for therapeutic applications, one would typically select a conjugate that produced no significant cytotoxic effects at concentrations below about 20 µM in normal cells. Testing the ability of a candidate compound to inhibit telomerase in cell-based assays is also routine, and is described in WO 01/18015, Herbert et al., Oncogene, 21: 638-642, 2002; Gryaznov et al., Nucleosides, Nucleotides and Nucleic Acids, 20: 401-410, 2001; Izbika et al., Cancer Res., 59:639-644, 1999; Shammas & Corey Oncogene 18: 6191-6200, 1999; and Pitts & Corey, Porc. Nat'l. Acad. Sci. USA, 95:11549-11554, 1998.

Confirmation of the ability of an oligonucleotide conjugate to inhibit tumor cell growth *in vivo* can be obtained using established xenograft models of human tumors, in which the test conjugate is administered either directly to the tumor site or systemically, and the growth of the tumor is followed by physical measurement. Animals treated with oligonucleotide conjugates of the invention are expected to have tumor masses that, on average, may increase for a period following the initial dosing, but will begin to shrink in mass with continuing treatment. In contrast, untreated control mice are expected to have tumor masses that continue to increase. Examples of such xenograft models in screening for cancer therapeutics are described in Scorski et al., Proc. Nat'l. Acad. Sci. USA, 94: 3966-3971, 1997; and Damm et a., EMBO J., 20:6958-6968, 2001.

### C. Formulation of Oligonucleotide Conjugates

The present invention provides oligonucleotide conjugates that can specifically and potently inhibit telomerase activity, and which may therefore be used to inhibit the proliferation of telomerase-positive cells, such as tumor cells. A very wide variety of cancer cells have been shown to be telomerase-positive, including cells from cancer of the skin, connective tissue, adipose, breast, lung, stomach, pancreas, ovary, cervix, uterus, kidney, bladder, colon, prostate, central nervous system (CNS), retina and circulating tumors (such as leukemia and lymphoma).

Accordingly, the oligonucleotide conjugates provided herein may provide a broadly useful in treating a wide range of malignancies. More importantly, the oligonucleotide conjugates of the present invention can be effective in providing treatments that discriminate between malignant and normal cells to a high degree, avoiding many of the deleterious side-effects present with most current chemotherapeutic regimes which rely on agents that kill dividing cells indiscriminately.

For therapeutic application, an oligonucleotide conjugate of the invention will be formulated in a therapeutically effective amount with a pharmaceutically acceptable carrier. One or more oligonucleotide conjugates (having different base sequences or linkages) may be included in any given formulation. The pharmaceutical carrier may be solid or liquid. Liquid carriers can be used in the preparation of solutions, emulsions, suspensions and pressurized compositions. The modified oligonucleotides are dissolved or suspended in a pharmaceutically acceptable liquid excipient. Suitable examples of liquid carriers for parenteral administration of the oligonucleotides preparations include water (partially containing additives, e.g., cellulose derivatives, preferably sodium carboxymethyl cellulose solution), alcohols (including monohydric alcohols and polyhydric alcohols, e.g., glycols) and their derivatives, and oils (e.g., fractionated coconut oil and arachis oil). The liquid carrier can contain other suitable pharmaceutical additives including, but not limited to, the following: solubilizers, suspending agents, emulsifiers, buffers, thickening agents, colors, viscosity regulators, preservatives, stabilizers and osmolarity regulators.

For parenteral administration of the oligonucleotides, the carrier can also be an oily ester such as ethyl oleate and isopropyl myristate. Sterile carriers are useful in sterile liquid form compositions for parenteral administration.

Sterile liquid pharmaceutical compositions, solutions or suspensions can be utilized by, for example, intraperitoneal injection, subcutaneous injection, intravenously, or topically. The oligonucleotides can also be administered intravascularly or via a vascular stent.

The liquid carrier for pressurized compositions can be halogenated hydrocarbon or other pharmaceutically acceptable propellant. Such pressurized compositions may also be lipid encapsulated for delivery via inhalation. For administration by intranasal or intrabronchial inhalation or insufflation, the oligonucleotides may be formulated into an aqueous or partially aqueous solution, which can then be utilized in the form of an aerosol.

The oligonucleotide conjugates may be administered topically as a solution, cream, or lotion, by formulation with pharmaceutically acceptable vehicles containing the active compound.

The pharmaceutical compositions of this invention may be orally administered in any acceptable dosage including, but not limited to, formulations in capsules, tablets, powders or granules, and as suspensions or solutions in water or non-aqueous media. Pharmaceutical compositions and/or formulations comprising the oligonucleotides of the present invention may include carriers, lubricants, diluents, thickeners, flavoring agents, emulsifiers, dispersing aids or binders. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Pharmaceutical compositions and/or formulations comprising the oligonucleotides of the present invention may also include cellular and tissue penetration enhancers. Penetration enhancers may include, for example, fatty acids, bile salts, chelating agents, surfactants and non-surfactants (Lee et al., Critical Reviews in Therapeutic Drug Carrier Systems, 1991, 8, 91-192; Muranishi, Critical Reviews in Therapeutic Drug Carrier Systems, 1990, 7, 1-33). One or more penetration enhancers may be included. Fatty acids and their derivatives which act as penetration enhancers include, for example, oleic acid, lauric acid, capric acid, myristic acid, palmitic acid, stearic acid, linoleic acid, linolenic acid, dicaprate, tricaprate, recinleate, monoolein (a.k.a. 1-monooleoyl-rac-glycerol), dilaurin, caprylic acid, arichidonic acid, glyceryl 1-monocaprate, 1-dodecylazacycloheptan-2-one, acylcarnitines, acylcholines, mono- and diglycerides and physiologically acceptable salts thereof (i.e., oleate, laurate, caprate, myristate, palmitate, stearate, linoleate, etc.). Examples of some presently preferred fatty acids are sodium caprate and sodium laurate, used singly or in combination at concentrations of about 0.5 to 5%.

Complex formulations comprising one or more penetration enhancers may be used. For example, bile salts may be used in combination with fatty acids to make complex formulations. Preferred combinations include chenodeoxycholic acid (CDCA), generally used at concentrations of about 0.5 to 2%, combined with sodium caprate or sodium laurate, generally used at concentrations of about 0.5 to 5%.

Pharmaceutical compositions and/or formulations comprising the oligonucleotides of the present invention may also include chelating agents, surfactants and non-surfactants. Chelating agents include, but are not limited to, disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of beta-diketones (enamines). Surfactants include, for example, sodium lauryl sulfate, polyoxyethylene-9-lauryl ether and polyoxyethylene-20-cetyl ether; and perfluorochemical emulsions, such as FC-43. Non-surfactants include, for example, unsaturated cyclic ureas, 1-alkyl- and I-alkenylazacyclo-alkanone derivatives, and non-steroidal anti-inflammatory agents such as diclofenac sodium, indomethacin and phenylbutazone.

The oligonucleotide conjugates may be administered in liposome carriers. The use of liposomes to facilitate cellular uptake is described, for example, in U.S. Patent No. 4,897,355 and U.S. Patent No. 4,394,448. Numerous publications describe the formulation and preparation of liposomes.

The dosage requirements for treatment with the oligonucleotide conjugates vary with the particular compositions employed, the route of administration, the severity of the symptoms presented, the form of the oligonucleotides and the particular subject being treated.

For use as an active ingredient in a pharmaceutical preparation, an oligonucleotide of this invention is generally purified away from other reactive or potentially immunogenic components present in the mixture in which they are prepared. Typically, each active ingredient is provided in at least about 90% homogeneity, and more preferably 95% or 99% homogeneity, as determined by functional assay, chromatography, or gel electrophoresis. The active ingredient is then compounded into a medicament in accordance with generally accepted procedures for the preparation of pharmaceutical preparations.

Pharmaceutical compositions of the invention can be administered to a subject in a formulation and in an amount effective to achieve a clinically desirable result. For the treatment of cancer, desirable results include reduction in tumor mass (as determined by palpation or imaging; e.g., by radiography, CAT scan, or MRI), reduction in the rate of tumor growth, reduction in the rate of metastasis formation (as determined e.g., by histochemical analysis of biopsy specimens), reduction in biochemical markers (including general markers such as ESR, and tumor-specific markers such as serum PSA), and improvement in quality of life (as determined by clinical assessment, e.g., Kamofsky score).

The amount of oligonucleotide per dose and the number of doses required to achieve such effects can be determined empirically using *in vitro* tests and animal models. An appropriate range for testing can be estimated from the 50% inhibitory concentration determined with isolated telomerase or cultured cells. Preparations of isolated telomerase can be obtained according to U.S. Patent 5,968,506. Typically, the formulation and route of administration will provide a local concentration at the disease site of between 1 µM and I nM of the oligonucleotide conjugate.

In general, the oligonucleotide conjugates are administered at a concentration that affords effective results without causing any harmful or deleterious side effects (e.g., an effective amount). Such a concentration can be achieved by administration of either a single unit dose, or by the administration of the dose divided into convenient subunits at suitable intervals throughout the day.

### Example 1

### General Methods

³¹P NMR spectra were obtained on a Varian 400 Mhz spectrometer. ³¹P NMR spectra were referenced against 85% aqueous phosphoric acid. Anion exchange HPLC was performed using a Dionex DX 500 Chromatography System, with a Pharmacia Biotech Mono Q HR 5/5 or 10/16 ion exchange columns. Mass spectral analysis was performed by Mass Consortium, San Diego, CA. MALDI-TOF analysis of oligonucleotides was obtained using a PerSpective Biosystems Voyager Elite mass spectrometer with delayed extraction. Thermal dissociation experiments were conducted on a Cary Bio 100 UV-Vis spectrometer.

All reactions were carried out in oven dried glassware under a nitrogen atmosphere unless otherwise stated. Commercially available DNA synthesis reagents were purchased from Glen Research (Sterling, VA). Anhydrous pyridine, toluene, dichloromethane, diisopropylethyl amine, triethylamine, acetic anhydride, 1,2-dichloroethane, and dioxane were purchased from Aldrich (Milwaukee, WI).

All non-thio-phosphoramidate oligonucleotides were synthesized on an ABI 392 or 394 DNA synthesizer using standard protocols for the phosphoramidite based coupling approach (Caruthers, Acc. Chem. Res., 24:278-284, 1991). The chain assembly cycle for the synthesis of oligonucleotide phosphoramidates was the following: (i) detritylation, 3% trichloroaceticacid in dichloromethane, 1 min; (ii) coupling, 0.1 M phosphoramidite and 0.45 M tetrazole in acetonitrile, 10 min; (iii) capping, 0.5 M isobutyic anhydride in THF/lutidine, 1/1, v/v, 15 sec; and (iv) oxidation, 0.1 M iodine in THF/pyridine/water, 10/10/1, v/v/v, 30 sec.

Chemical steps within the cycle were followed by acetonitrile washing and flushing with dry argon for 0.2-0.4 min. Cleavage from the support and removal of base and phosphoramidate protecting groups was achieved by treatment with ammonia/EtOH, 3/1, v/v, for 6 h at 55°C. The oligonucleotides were concentrated to dryness *in vacuo* after which the 2'-t-butyldimethylsilyl groups were removed (if present) by treatment with 1M TBAF in THF for 4-16 h at 25°C. The reaction mixtures were diluted with water and filtered through a 0.45 nylon acrodisc (from Gelman Sciences, Ann Arbor, MI). Oligonucleotides were then analyzed and purified by IE HPLC and finally desalted using gel filtration on a Pharmacia NAP-5 or NAP-25 column. Gradient conditions for IE HPLC: solvent A (10 mM NaOH), solvent B (10 mM NaOH and 1.5 M NaCl); solvent A for 3 min then a linear gradient 0-80% solvent B within 50 min.

### Example 2

### Synthesis of Oligoribonucleotide N3' → P5' Phosphoramidates

It was previously reported that homopurine and homopyrimidine oligoribonucleotide N3'→P5' phosphoramidates could be efficiently assembled on a solid phase support using a phosphoramidite transfer reaction (Gryaznov, et al. (1998) Nucleic Acids Res., 26:4160-4167). We found that this methodology works equally well for the synthesis of heterobased phosphoramidate oligoribonucleotides containing all four natural bases as well as thymidine and 2,4-diaminopurine (D). Each of the prepared oligoribonucleotide N3'→P5' phosphoramidites were synthesized starting from the 5'-end using a support-bound 2'-deoxy-3'-aminonucleoside as the 5'-terminal residue. Coupling steps involved exchange of the diisopropylamino group of the approaching 5'-*O*-phosphoramidite for the 3'-amino group of the support bound nucleoside. Standard RNA synthesis coupling times (10 min) and activator (1H-tetrazole) were used for each synthetic cycle. Unreacted 3'-amino groups were then capped with isobutyric anhydride, after which oxidation of the internucleotide phosphoramidite diester linkage into the phosphoramidate group was carried out with aqueous iodine. Subsequent detritylation of the 3'-amino group of the added residue enabled additional chain elongation steps to be repeated for the construction of the desired oligoribonucleotide phosphoramidates. The resin bound compounds were then deprotected and cleaved from the support by treatment with ammonia/ethanol solution. Removal of 2'-O-t-butyldimethylsilyl groups was accomplished using 1M TBAF in THF after which the fully deprotected oligoribonucleotide phosphoramidates were analyzed and isolated using IE. The mixed base 9-13-mer oligoribonucleotide phosphoramidates were synthesized with step-wise coupling yields of about 96-98%, as judged by MMTr assays. The oligonucleotides were characterized by both ³¹P NMR and MALDI-TOF mass spectra analysis.

### Example 3

### Synthesis of Oligonucleotide N3'→P5' Thio-phosphoramidates

Oligonucleotide N3'→P5' thio-phosphoramidates were prepared by the amidite transfer reaction on an ABI 394 synthesizer. The fully protected monomer building blocks were 3'-aminotrityl nucleoside-5' -(2-cyanoethyl-N,N-diisopropyl) phosphoramidite where nucleoside is 3'-deoxythymidine, 2',3'-dideoxy-N²-isobutyryl-guanosine, 2',3'-dideoxy-N⁶-benzoyl-adenosine or 2',3'-dideoxy-N⁴-benzoyl-cytidine. 5'-Succinyl-3'-aminotrityl-2',3'-dideoxy nucleosides were coupled with an amino group containing long chain controlled pore glass (LCAA-CPG) and used as the solid support. The synthesis was performed in the direction of 5' to 3'. The following protocol was used for the assembly of oligonucleotide N3'→P5' thio-phosphoramidates: (i) detritylation, 3% dichloroacetic acid in dichloromethane; (ii) coupling, 0.1 M phosphoramidite and 0.45 M tetrazole in acetonitrile, 25 sec; (iii) capping, isobutyric anhydride/2,6-lutidine/THF 1/1/8 v/v/v as Cap A and standard Cap B solution; (iv) sulfurization, 15% S₈ in carbon disulfide containing 1% triethylamine, 1 min. Before and after the sulfurization step the column was washed with neat carbon disulfide to prevent elemental sulfur precipitation. The oligonucleotide thio-phosphoramidates were cleaved from the solid support and deprotected with concentrated aqueous ammonia. The compounds were analyzed and purified by HPLC. Ion exchange (IE) HPLC was performed using DIONEX DNAPac™ ion exchange column at pH 12 (10 mM NaOH) with a 1%/min linear gradient of 10 mM NaOH in 1.5 M NaCl and a flow rate of 1 ml/min. The products were desalted on Sephadex NAP-5 gel filtration columns (Pharmacia) and lyophilized *in vacuo.* ³¹P NMR experiments were performed in deuterium oxide to analyze the extent of sulfurization analysis (³¹P NMR δ, ppm 58, 60 broad signals for Rp-, Sp- isomers).

Oligonucleotide thio-phosphoramidate 5'-GTTAGGGTTAG-3' (SEQ ID NO.: 14) was synthesized the following way: An ABI Model 394 synthesizer was set up with 0.1M solutions of 3'-tritylamino-2',3'-dideoxy-N⁶-benzoyl-adenosine (N²-isobutyryl-guanosine, and thymidine) 5'-(2-cyanoethyl-N,N-diisopropyl)phosphoramidites. The reagent bottle of station #10 was filled with neat carbon disulfide and reagent bottle #15 was filled with a solution of 15% S₈ in carbon disulfide containing 1% triethylamine. As the activator the commercially available 0.45 M solution of tetrazole in acetonitrile was used. Cap A solution (station #11) was replaced by tetrahydrofuran/isobutyric anhydride/2,6-lutidine 8/1/1 v/v/v solution. Cap B was also the commercially available reagent. A new function was created to deliver carbon disulfide from station #10 to the column. The default sulfur synthesis cycle was modified the following way: sulfurization time was set at 1 min., and before and after sulfurization carbon disulfide was delivered to the column for 20 s. The synthesis column was filled with 1 µmole solid support N²-isobutyryl-3'-(trityl)amino-2',3'-dideoxyguanosine-5'-succinyl-loaded CPG (controlled pore glass). The sequence of the compound was programmed as GATTGGGATTG (5'→3') (SEQ ID NO: 15). The trityl group was removed at end of the synthesis and the column was washed manually with carbon disulfide and acetonitrile. The solid support was removed from the column and treated with 1 ml concentrated aqueous ammonia at 55°C for 6 hr in a tightly closed glass vial. After filtration most of the ammonia was evaporated and the remaining solution was desalted using Sephadex™ NAP-5 gel filtration columns (Pharmacia) followed by lyophilization *in vacuo.* The product was analyzed and purified as described above.

### Example 4

### Conjugation of Fluorescein to 3'- amino or 5'-amino oligonucleotides:

The reaction was conducted in 1.5 mL Eppendorff test tub, were ~10-20 OD of an oligonucleotide with free amino group was dissolved in 100 uL of 0.1 sodium-bicarbonate buffer, pH 8, and 50 uL of DMSO. Then ~1 mg of FITC (from Aldrich) was added to the solution, and the reaction mixture was shaken by vortexing for 1-2 minutes. The reaction mixture was heated to 55 °C for 30 minutes, and than left over night at room temperature in darkness. The oligonucleotide product was precipitated in the same test tube by the addition of 1.2 mL of EtOH, at -18 °C for 1 hour. The precipitate was separated from supernatant, and repricipitated twice with EtOH from 100 uL of 1 M NaCl.

The obtained oligonucleotide conjugate was then analyzed and purified if needed by RP HPLC. Yields of the oligonucletide conjugates with FITC were 85-95%.

### Example 5

### Preparation of Affinity Purified Extract Having Telomerase Activity

Extracts used for screening telomerase inhibitors were routinely prepared from 293 cells over-expressing the protein catalytic subunit of telomerase (hTERT). These cells were found to have 2-5 fold more telomerase activity than parental 293 cells. 200 ml of packed cells (harvested from about 100 liters of culture) were resuspended in an equal volume of hypotonic buffer (10 mM Hepes pH 7.9, 1 mM MgCl₂, 1 mM DTT, 20 mM KCl, 1 mM PMSF) and lysed using a dounce homogenizer. The glycerol concentration was adjusted to 10% and NaCl was slowly added to give a final concentration of 0.3 M. The lysed cells were stirred for 30 min and then pelleted at 100,000 x g for 1 hr. Solid ammonium sulfate was added to the S100 supernatant to reach 42% saturation. The material was centrifuged; the pellet was resuspended in one fifth of the original volume and dialyzed against Buffer 'A' containing 50 mM NaCl. After dialysis the extract was centrifuged for 30 min at 25,000 x g. Prior to affinity chromatography, Triton X-100T™ (0.5 %), KCl (0.3 M) and tRNA (50 µg/ml) were added. Affinity oligonucleotide (5'-biotinTEG-biotinTEG-biotinTEG-GTA GAC CTG TTA CCA guu agg guu ag 3' [SEQ ID NO: 16]; lower case represents 2' O-methyl ribonucleotides and upper case represents deoxynucleotides) was added to the extract (1 nmol per 10 ml of extract). After an incubation of 10 min at 30 °C, Neutravidin beads (Pierce; 250 µl of a 50% suspension) were added and the mixture was rotated overnight at 4 °C. The beads were pelleted and washed three times with Buffer 'B' containing 0.3 M KCl, twice with Buffer 'B' containing 0.6 M KCl, and twice more with Buffer B containing 0.3 M KCl. Telomerase was eluted in Buffer 'B' containing 0.3 M KCl, 0.15% Triton X-100™ and a 2.5 molar excess of displacement oligonucleotide (5'-CTA ACC CTA ACT GGT AAC AGG TCT AC-3' [SEQ ID NO: 17] at 0.5 ml per 125 µl of packed Neutravidin beads) for 30 min. at room temperature. A second elution was performed and pooled with the first. Purified extracts typically had specific activities of 10 fmol nucleotides incorporated/min/µl extract, or 200 nucleotides/min/mg total protein.

| **Buffer 'A'** | | **Buffer 'B'** |
|---|---|---|
| 20 mM Hepes pH 7.9 | | 20 mM Hepes pH 7.9 |
| 1 mM MgCl2 | | 1 mM EDTA |
| 1 mM DTT | | 1 mM DTT |
| 1 mM EGTA | | 10% glycerol |
| 10 % glycerol | | 0.5 Triton X-100™ |

### Example 6

### Telomerase Inhibition by Oligonucleotide Conjugates

### Bio-Tel FlashPlate Assay

A suitable assay for detection and/or measurement of telomerase activity is based on a measurement of the addition of TTAGGG (SEQ ID NO. 8) telomeric repeats to a biotinylated telomerase substrate primer; a reaction catalyzed by telomerase. The biotinylated products are captured in streptavidin-coated microtiter plates. An oligonucleotide probe complementary to 3.5 telomere repeats labeled with ³³P is used for measuring telomerase products, as described below. Unbound probe is removed by washing and the amount of probe annealing to the captured telomerase products is determined by scintillation counting.

The data provided in Table 1 below were obtained by performing the assay as described below:
1. Oligonucleotide conjugates were stored as concentrated stocks and dissolved in PBS.
2. For testing, the oligonucleotide conjugates were diluted to a 15X working stock in PBS and 2 µl was dispensed into two wells of a 96-well microtiter dish (assayed in duplicate).
3. Telomerase extract was diluted to a specific activity of 0.04 - 0.09 fmol dNTP incorporated/min./µl in Telomerase Dilution Buffer and 18 µl added to each sample well to preincubate with compound for 30 minutes at room temperature.
4. The telomerase reaction was initiated by addition of 10 µl Master Mix to the wells containing telomerase extract and oligonucleotide compound being tested. The plates were sealed and incubated at 37°C for 90 min.
5. The reaction was stopped by the addition of 10 µl HCS.
6. 25 µl of the reaction mixture was transferred to a 96-well streptavidin-coated FlashPlate™ (NEN) and incubated for 2 hours at room temperature with mild agitation.
7. The wells were washed three times with 180 µl 2X SSC without any incubation.
8. The amount of probe annealed to biotinylated telomerase products was detected in a scintillation counter.

### Buffers:

### Telomerase Dilution Buffer

50 mM Tris-acetate, pH 8.2
1 mM DTT
1 mM EGTA
1 mM MgCl₂
830 nM BSA

### Master Mix (MM)

50 mM Tris-acetate, pH 8.2
1 mM DTT
1 mM EGTA
1 mM MgCl₂
150 mM K acetate
10 µM dATP
20 µM dGTP
120 µM dTTP
100 nM biotinylated primer (5'-biotin-AATCCGTCGAGCAGAGTT-3') [SEQ ID NO.: 18]
5.4 nM labeled probe [5'-CCCTAACCCTAACCCTAACCC-(³³P) A₁-₅₀-3'] [SEQ ID NO: 19]; specific activity approximately 10⁹ cpm/µg or higher

### Hybridization Capture Solution (HCS)

12X SSC (1X = 150 mM NaCl/30 mM Na₃Citrate)
40 mM EDTA
40 mM Tris-HCl, pH 7.0

Using the foregoing assay, various oligonucleotide conjugates were tested, and the data is presented in Table 1. As can be seen, the addition of the aromatic group to the oligonucleotides typically enhanced the inhibitory activity of the oligonucleotide, sometimes by by several orders of magnitude.

**TABLE 1.**

| **Evaluation Of Oligonucleotide Conjugates As Telomerase Inhibitors** | | | |
|---|---|---|---|
| **Oligonucleotide Conjugate** | | **(IC₅₀** *µ***M)** | |
| | | **NP** | **NPS** |
| GGGTTAG (unconjugated) | | 0.6 | 0.008 |
| GGGTTAG-L1-F | | 0.08 | 0.002 |
| GGGTTAG-L2-F | | 0.06 | 0.003 |
| | | | |
| GTTAGG (unconjugated) | | 0.47 | 0.12 |
| GTTAGG-L1-F | | 0.2 | 0.016 |
| GTTAGG-L2-F | | 1.5 | 0.015 |
| | | | |
| | TTAGGG (unconjugated) | 11.0 | 1.2 |
| | TTAGGG-L1-F | 0.034 | 0.003 |
| | TTAGGG-L2-F | 2.0 | 0.009 |
| F-L2-TTAGGG | | | 0.007 |
| F-L2-TTAGGG-L1-F | | 0.005 | 0.005 |
| | TTAGGG-L2-Tr | 11.5 | |
| | TTAGGG-L1-Tr | 0.4 | 0.04 |
| | TTAGGG-L1-X | 0.14 | 0.09 |
| A-L2-TTAGGG | | 1.0 | 0.06 |
| A-L2-TTAGGG-L1-F | | 0.022 | |
| | | | |
| AGGG (unconjugated) | | 100, >1 | 0.087, 0.05, 0.03 |
| AGGG-L1-F | | >1 | 0.018 |
| | | | |
| GGGTTAG (unconjugated) | | 0.1, 1, 0.6* | 0.009, 0.005, 0.01 |
| GGGTTAG-L1-F | | 0.1338, 0.0762, 0.196 | 0.002, 0.005, 0.005 |
| GGGTTAG-L2-F | | 0.059 | 0.003, 0.005 |
| GGGTTAG-L1-Tr | | 0.436, 0.933 | 0.029, 0.077 |
| | | | |
| TGAGT (unconjugated) | | 0.399 | 0.036 |
| TGAGT-L1-F | | 0.173,0.272 | 0.010, 0.029 |
| | | | |
| GTAGGT | | 0.098, 0.162 | 0.140, 0.117 |
| GTAGGT-L1-F | | 0.0156, 0.032, 0.015 | 0.099, 0.035, 0.033 |
| | | | |

### Key:

NP indicates that the oligonucleotide has N3'→P5' phosphoramidate internucleoside linkages
NPS indicates that the oligonucleotide has N3'→P5' thio-phosphoramidate internucleoside linkages.
L1: Type 1 linker (thiourea)
L2: Type 2 linker (C5 or C6)
F: fluorescein.
Tr: N,N'-tetramethylrhodamine
A: Acridine

### Example 7

### Anti-Tumor Activity of Oligonucleotide Conjugates

### Cell Assays

### a. Inhibition of telomerase activity in cells and inhibition of tumor cell growth

The following is a description of a general cell assay method that may be used to determine the effect of the test conjugates on tumor cell growth. Colonies of human breast epithelial cells (spontaneously immortalized) are prepared using standard methods and materials. Colonies are prepared by seeding 15-centimeter dishes with about 10⁶ cells in each dish. The dishes are incubated to allow the cell colonies to grow to about 80% confluence, at which time each of the colonies are divided into two groups. One group was exposed to a subacute dose of thio-phosphoramidate polynucleotide conjugate of experiment 9 at a predetermined concentration (e.g., between about 100 nM and about 20 µM) for a period of about 4-8 hours after plating following the split. The second group of cells are similarly exposed to an unconjugated control oligonucleotide.

Each group of cells is then allowed to continue to divide, and the groups are split evenly again (near confluence). The same number of cells are e seeded for continued growth. The test thio-phosphoramidate oligonucleotide conjugate or control oligonucleotide is added every fourth day to the samples at the same concentration delivered initially. In certain experiments, the cells may also be treated with an oligonucleotide uptake enhancer, such as FuGENE6™ (Roche). Reduction of telomerase activity associated with the oligonucleotide treatment is determined by TRAP assay.

In addition, telomere lengths in the treated cells may be determined by digesting the DNA of the cell samples using restriction enzymes specific for sequences other than the repetitive T₂AG₃ sequence of human telomeres (TRF analysis). The digested DNA is separated by size using standard techniques of gel electrophoresis to determine the lengths of the telomeric repeats, which appear, after probing with a telomere DNA probe, on the gel as a smear of high-molecular weight DNA (approximately 2 Kb-15 Kb).

These approaches were used to determine the effect of treatment with the conjugate TTAGGG-L1-F on HME50 and Caki-1 cells. IC₅₀ values in the range of ca. 5 µmole (in the range of 1-20 µmole) were obtained in the absence of an uptake enhancer. When cells were incubated with this conjugate in the presence of FuGENE6 they underwent cell crisis and apoptosis after approximately 15 days of treatment. Growth of control cells was not affected.

### b. Specificity

The short oligonucleotide conjugates of the invention are screened for activity (IC₅₀) against telomerase and other enzymes known to have RNA components by performing hybridization tests or enzyme inhibition assays using standard techniques. Oligonucleotides having lower IC₅₀ values for telomerase as compared to the IC₅₀ values toward the other enzymes being screened are said to possess specificity for telomerase.

### c. Cytotoxicity

The cell death (XTT) assay for cytotoxicity are performed using HME50-5E, Caki-1, A431, ACHN, and A549 cell types. The cell lines used in the assay are exposed to one of the short oligonucleotide conjugates for 72 hours at concentrations ranging from about 1 µM to about 100 µM in the presence and absence of lipids. During this period, the optical density (OD) of the samples is determined for light at 540 nanometers (nm). The IC₅₀ values obtained for the various cell types are generally less than 1 µM. Thus, no significant cytotoxic effects are expected to be observed at concentrations less than about 100 µM. It will be appreciated that other tumor cells lines such as the ovarian tumor cell lines OVCAR-5 and SK-OV-3 can be used to determine cytotoxicity in addition to control cell lines such as normal human BJ cells. Other assays for cytotoxicity such as the MTT assay (see Berridge et al., Biochemica 4:14-19, 1996) and the alamarBlue™ assay (U.S. Patent No. 5,501,959) can be used as well.

Preferably, to observe any telomerase inhibiting effects the oligonucleotide conjugates should be administered at a concentration below the level of cytotoxicity. Nevertheless, since the effectiveness of many cancer chemotherapeutics derives from their cytotoxic effects, it is within the scope of the present invention that the oligonucleotide conjugates of the present invention be administered at any dose for which chemotherapeutic effects are observed.

### In vivo Animal Studies

A human tumor xenograft model in which OVCAR-5 tumor cells are grafted into nude mice can be constructed using standard techniques and materials. The mice are divided into two groups. One group is treated intraperitoneally with a short oligonucleotide conjugates of the invention. The other group is treated with a control comprising a mixture of phosphate buffer solution (PBS) and an oligonucleotide complementary with telomerase RNA but has at least a one base mismatch with the sequence of telomerase RNA. The average tumor mass for mice in each group is determined periodically following the xenograft using standard methods and materials.

In the group treated with a oligonucleotide conjugates of the invention, the average tumor mass is expected to increase following the initial treatment for a period of time, after which time the tumor mass is expected to stabilize and then begin to decline. Tumor masses in the control group are expected to increase throughout the study. Thus, the oligonucleotide conjugates of the invention are expected to lessen dramatically the rate of tumor growth and ultimately induce reduction in tumor size and elimination of the tumor.

Thus, the present invention provides novel oligonucleotide conjugates and methods for inhibiting telomerase activity and treating disease states in which telomerase activity has deleterious effects, especially cancer. The oligonucleotide conjugates of the invention provide a highly selective and effective treatment for malignant cells that require telomerase activity to remain immortal; yet, without affecting non-malignant cells.

## Claims

1. A compound comprising the structure:
(A-L-)ₙ-O
wherein (A-L-) is independently selected from the group consisting of: 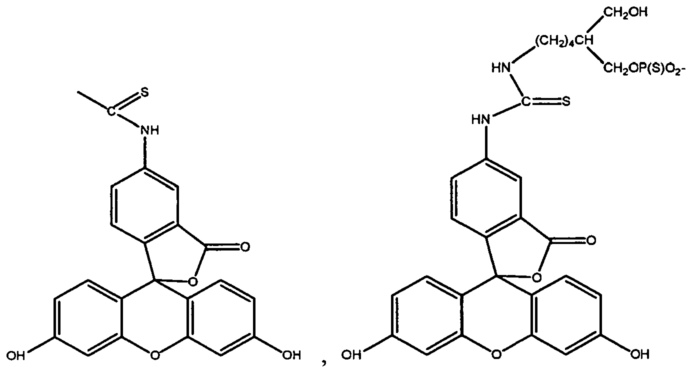 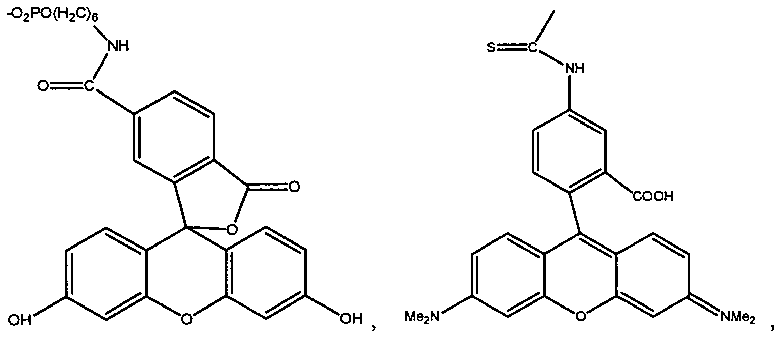 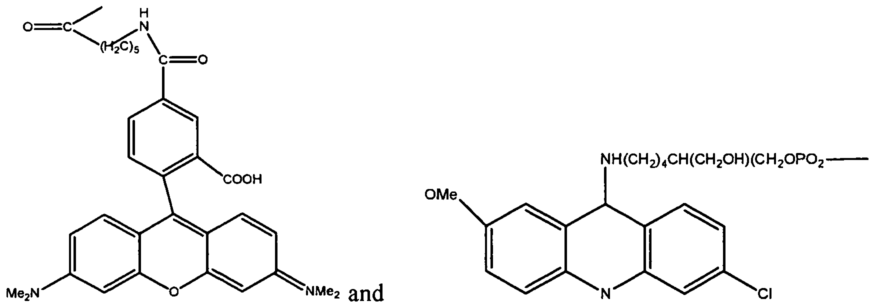
O is an oligonucleotide having a sequence selected from the group consisting of: TAGGGTTAGACAA; GTTAGGGTTAG; AGGGTTAG; GGGTTAG; GTTAGG; TTAGGG; AGGG; GGGTTA; TGAGTG; and GTAGGT comprising N3'→P5' phosphoramidate or N3'→P5' thiophosphoramidate intersubunit linkages;
n is an integer between 1 and m+1 where m is the total number of nucleosides in O, wherein the compound inhibits telomerase activity.

2. The compound according to claim 1 wherein n=1 and (A-L) group is covalently conjugated to either the 5' or the 3' terminus of the oligonucleotide O.

3. The compound according to claim 1 wherein n=2 and an (A-L) group is covalently conjugated to the 5' and the 3' terminus of the oligonucleotide O.

4. The compound according to the previous claims, wherein the (A-L) group covalently conjugated to the 3' terminus of the oligonucleotide O is

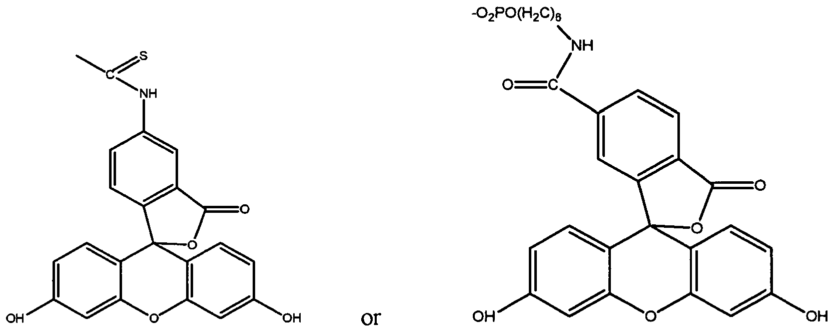

5. The compound of claims 1 to 3, wherein the (A-L) group covalently conjugated to the 3' terminus of the oligonucleotide O is

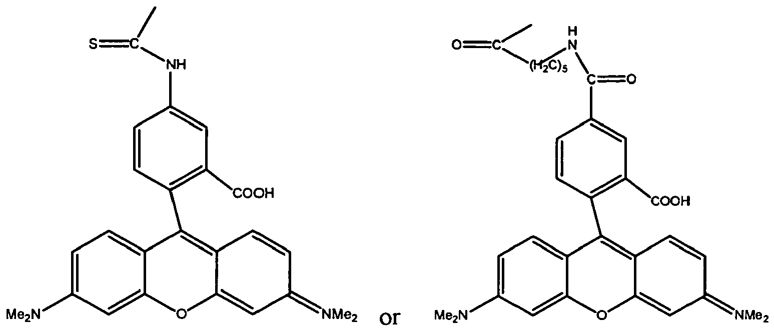

6. The compound of claims 1 to 3, wherein the (A-L) group covalently conjugated to the 5' terminus of the oligonucleotide O is

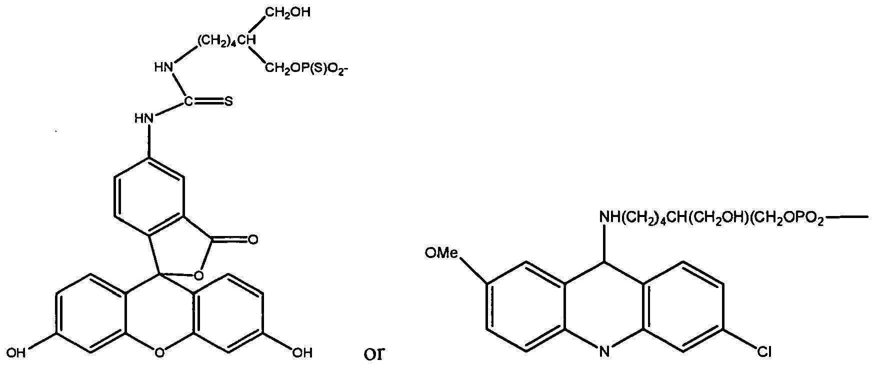

7. A compound according to any of claims 1 to 6 for use in inhibiting the activity of a telomerase enzyme.

8. A compound according to any one of claims 1 to 6 for use in medicine.

9. A pharmaceutical composition comprising a conjugate compound according to any one of claims 1 to 6 formulated in a pharmaceutically acceptable excipient.

## Patentansprüche

1. Verbindung, die folgende Struktur umfasst:
(A-L-)ₙ-O
wobei (A-L-) unabhängig ausgewählt ist aus der Gruppe bestehend aus: 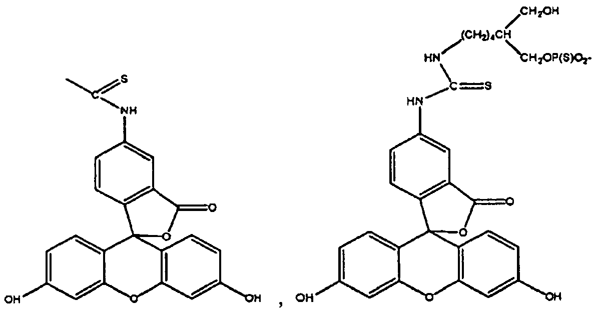 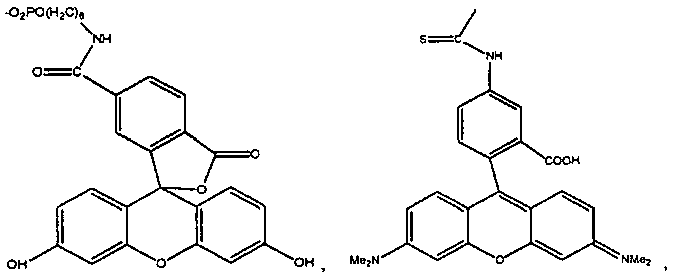 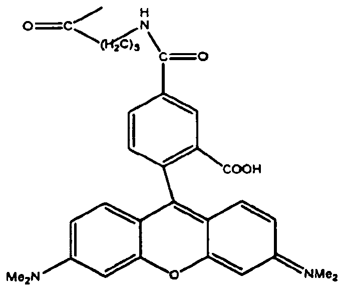 und 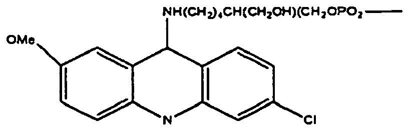
O ein Oligonukleotid mit einer Sequenz ausgewählt aus der Gruppe bestehend aus: TAGGGTTAGACAA; GTTAGGGTTAG; AGGGTTAG; GGGTTAG; GTTAGG; TTAGGG; AGGG; GGGTTA; TGAGTG und GTAGGT ist, welches N3'→P5' Phosphoramidat oder N3'→P5' Thiophosphoramidat Zwischen-Untereinheit-Verknüpfungen umfasst;
n eine ganze Zahl zwischen 1 und m+1 ist, wobei m die Gesamtanzahl der Nukleoside in O ist, wobei die Verbindung die Telomerase-Aktivität inhibiert.

2. Verbindung nach Anspruch 1, wobei n=1 und die (A-L)-Gruppe kovalent entweder an den 5'- oder den 3'-Terminus des Oligonukleotides O konjugiert ist.

3. Verbindung nach Anspruch 1, wobei n=2 und eine (A-L)-Gruppe kovalent an den 5'- und den 3'-Terminus des Oligonukleotides O konjugiert ist.

4. Verbindung nach den vorhergehenden Ansprüchen, wobei die (A-L)-Gruppe, die kovalent an den 3'-Terminus des Oligonukleotides O konjugiert ist, Folgende ist: 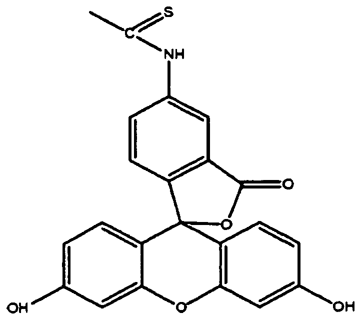 oder

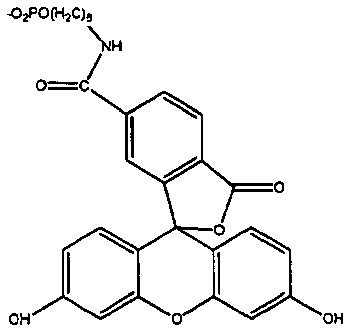

5. Verbindung nach Anspruch 1 bis 3, wobei die (A-L)-Gruppe, die kovalent an den 3'-Terminus des Oligonukleotides O gekoppelt ist, Folgende ist: 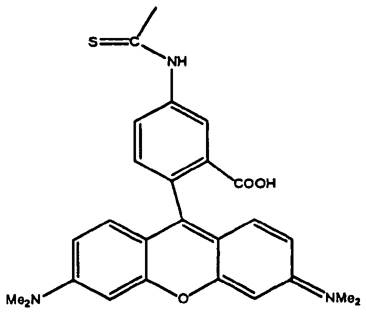 oder

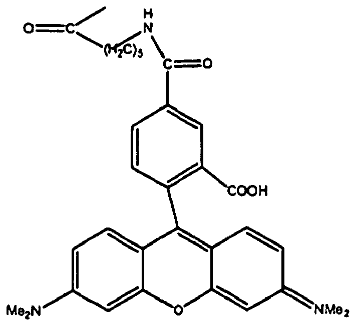

6. Verbindung nach Anspruch 1 bis 3, wobei die (A-L)-Gruppe, die kovalent an den 5'-Terminus des Oligonukleotides O gekoppelt ist, Folgende ist: 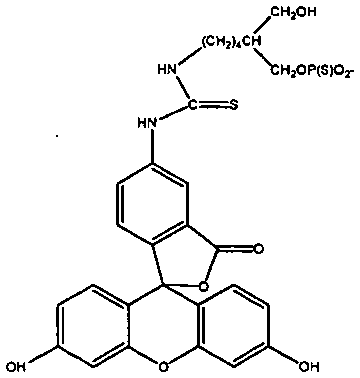 oder

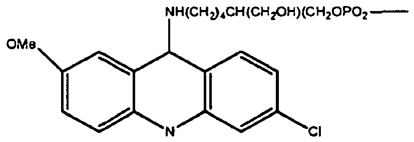

7. Verbindung nach einem der Ansprüche 1 bis 6 zur Verwendung beim Inhibieren der Aktivität eines Telomerase-Enzyms.

8. Verbindung nach einem der Ansprüche 1 bis 6 zur Verwendung in der Medizin.

9. Pharmazeutische Zusammensetzung, die eine konjugierte Verbindung nach einem der Ansprüche 1 bis 6 formuliert in einem pharmazeutisch akzeptablen Exzipienten umfasst.

## Revendications

1. Composé comprenant la structure :
(A - L -)ₙ-O
dans laquelle (A-L-) est choisi indépendamment dans le groupe constitué de : 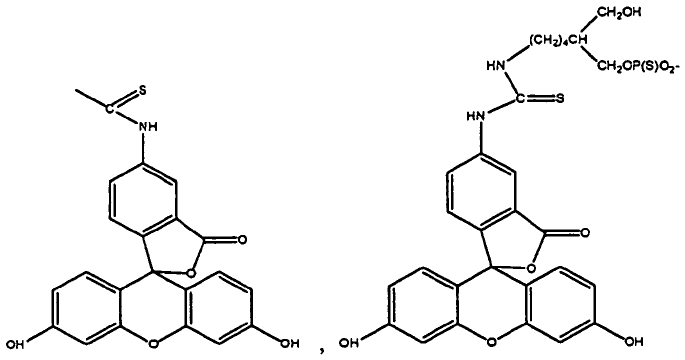 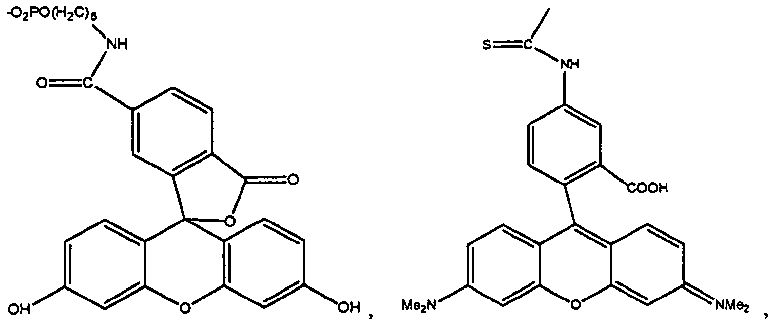 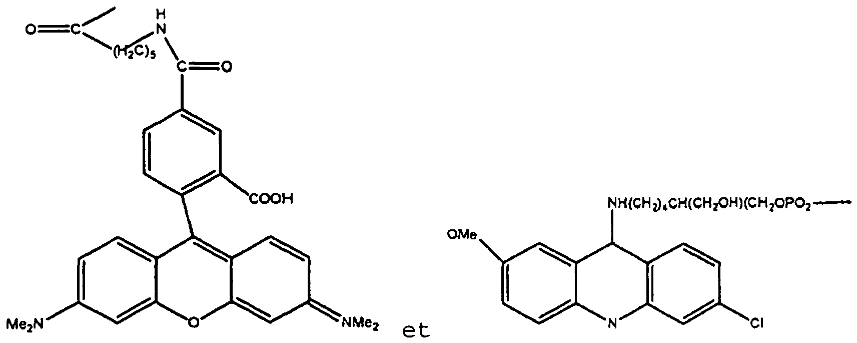
O est un oligonucléotide ayant une séquence choisie dans le groupe constitué de : TAGGGTTAGACAA ; GTTAGGGTTAG ; AGGGTTAG ; GGGTTAG ; GTTAGG ; TTAGGG ; AGGG ; GGGTTA ; TGAGTG ; et GTAGGT comprenant des liaisons inter-sous-unités phosphoramidate N3' → P5' ou thiophosphoramidate N3' → P5',
n est un nombre entier compris entre 1 et m+1 où m est le nombre total de nucléosides dans O,
dans lequel le composé inhibe l'activité de la télomérase.

2. Composé selon la revendication 1, dans lequel n = 1 et le groupe (A-L) est conjugué par covalence à l'extrémité 5' ou 3' de l'oligonucléotide O.

3. Composé selon la revendication 1, dans lequel n = 2 et le groupe (A-L) est conjugué par covalence à l'extrémité 5' ou 3' de l'oligonucléotide O.

4. Composé selon les revendications précédentes, dans lequel le groupe (A-L) conjugué par covalence à l'extrémité 3' de l'oligonucléotide O est

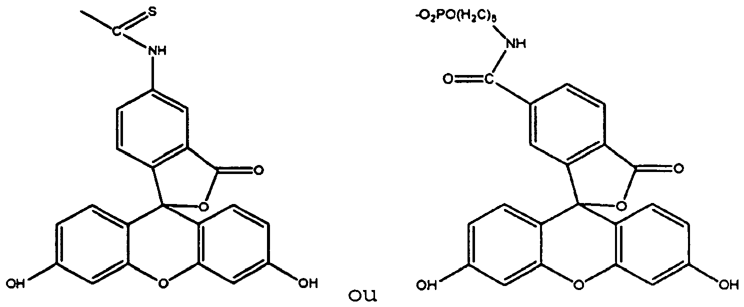

5. Composé selon les revendications 1 à 3, dans lequel le groupe (A-L) conjugué par covalence à l'extrémité 3' de l'oligonucléotide O est

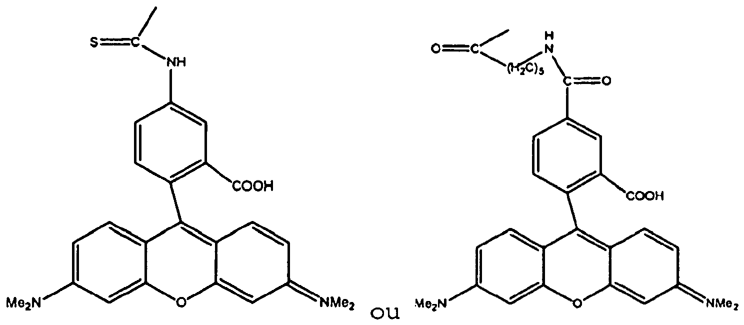

6. Composé selon les revendications 1 à 3, dans lequel le groupe (A-L) conjugué par covalence à l'extrémité 5' de l'oligonucléotide O est

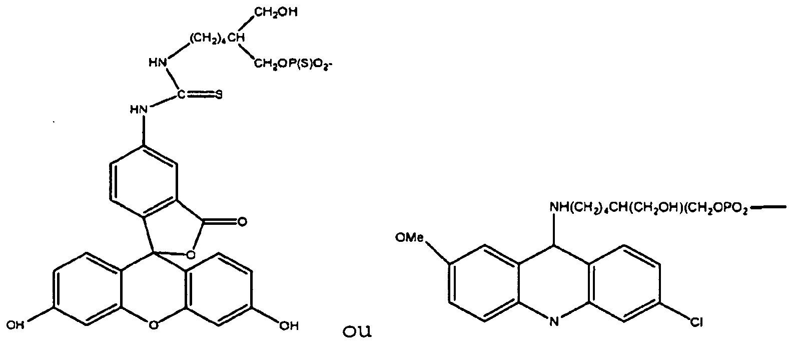

7. Composé selon l'une quelconque des revendications 1 à 6, destiné à une utilisation dans l'inhibition de l'activité d'une enzyme télomérase.

8. Composé selon l'une quelconque des revendications 1 à 6, destiné à une utilisation en médecine.

9. Composition pharmaceutique comprenant un composé conjugué selon l'une quelconque des revendications 1 à 6 formulé dans un excipient pharmaceutiquement acceptable.


### SEQUENCE LISTING

<110> Geron Corporation
Gryaznov, Sergei Pongracz, Krisztina
Tolman, Richard L.
Morin, Gregg B.
<120> oligonucleotide Conjugates
<130> 071/200PCT
<160> 19
<170> PatentIn version 3.1
<210> 1
   <211> 3
   <212> DNA
   <213> Artificial Sequence
<220>
   <223> oligonucleotide
<400> 1
   agg 3
<210> 2
   <211> 11
   <212> RNA
   <213> Homo sapiens
<220>
   <221> modified_base
   <222> (2) .. (2)
   <223> x
<220>
   <221> modified_base
   <222> (8) .. (8)
   <223> x
<400> 2
   cyaacccyaa c 11
<210> 3
   <211> 13
   <212> DNA
   <213> Artificial Sequence
<220>
   <223> oligonucleotide
<400> 3
   tagggttaga caa 13
<210> 4
   <211> 11
   <212> DNA
   <213> Artificial Sequence
<220>
   <223> oligonucleotide
<400> 4
   gttagggttag 11
<210> 5
   <211> 8
   <212> DNA
   <213> Artificial Sequence
<220>
   <223> oligonucleotide
<400> 5
   agggttag 8
<210> 6
   <211> 7
   <212> DNA
   <213> Artificial Sequence
<220>
   <223> oligonucleotide
<400> 6
   gggttag 7
<210> 7
   <211> 6
   <212> DNA
   <213> Artificial sequence
<220>
   <223> oligonucleotide
<400> 7
   gttagg 6
<210> 8
   <211> 6
   <212> DNA
   <213> Artificial sequence
<220>
   <223> oligonucleotide
<400> 8
   ttaggg 6
<210> 9
   <211> 3
   <212> DNA
   <213> Artificial sequence
<220>
   <223> oligonucleotide
<400> 9
   tta 3
<210> 10
   <211> 4
   <212> DNA
   <213> Artificial Sequence
<220>
   <223> oligonucleotide
<400> 10
   aggg 4
<210> 11
   <211> 6
   <212> DNA
   <213> Artificial Sequence
<220>
   <223> oligonucleotide
<400> 11
   gggtta 6
<210> 12
   <211> 6
   <212> DNA
   <213> Artificial Sequence
<220>
   <223> oligonucleotide
<400> 12
   tgagtg 6
<210> 13
   <211> 6
   <212> DNA
   <213> Artificial Sequence
<220>
   <223> oligonucleotide
<400> 13
   gtaggt 6
<210> 14
   <211> 11
   <212> DNA
   <213> Artificial sequence
<220>
   <223> oligonucleotide
<400> 14
   gttagggtta g 11
<210> 15
   <211> 11
   <212> DNA
   <213> Artificial sequence
<220>
   <223> oligonucleotide
<400> 15
   gattgggatt g 11
<210> 16
   <211> 27
   <212> DNA
   <213> Artificial
<220>
   <221> modified_base
   <222> (18)..(19)
   <223> x
<220>
   <221> modified_base
   <222> (24)..(25)
   <223> x
<220>
   <221> misc_feature
   <222> (1) .. (1)
   <223> N is (biotin TEG)3 label
<400> 16
   ngtagacctgttaccagyyagggyyag 27
<210> 17
   <211> 26
   <212> DNA
   <213> Artificial Sequence
<220>
   <223> oligonucleotide
<400> 17
   ctaaccctaactggtaacaggtctac 26
<210> 18
   <211> 18
   <212> DNA
   <213> Artificial sequence
<220>
   <223> oligonucleotide
<400> 18
   aatccgtcga gcagagtt 18
<210> 19
   <211> 22
   <212> DNA
   <213> Artificial sequence
<220>
   <223> oligonucleotide
<220>
   <221> misc_feature
   <222> (22)..(22)
   <223> 1 to 50 33P adenine nucleotide
<400> 19
   ccctaaccct aaccctaacc ca 22